(19) Europäisches Patentamt
European Patent Office
Office européen des brevets

(11) EP 0 477 444 B1

(12) **EUROPEAN PATENT SPECIFICATION**

(45) Date of publication and mention of the grant of the patent:
**08.04.1998 Bulletin 1998/15**

(51) Int Cl.[6]: **C08F 4/00**, C07C 211/62, C07D 263/12, C07C 255/19, C07D 217/26

(21) Application number: **90310566.6**

(22) Date of filing: **27.09.1990**

(54) **Carbanionic polymerisation initiators**

Carbanionische Polymerisationsinitiatoren

Initiateurs de polymérisation carbanioniques

(84) Designated Contracting States:
**BE DE FR GB IT NL**

(43) Date of publication of application:
**01.04.1992 Bulletin 1992/14**

(73) Proprietor: **Council of Scientific & Industrial Research**
**New Delhi 110001 (IN)**

(72) Inventors:
- **Bhaskaran, Durairaj**
  **Maharashtra (IN)**
- **Dhal. Pradeep Kumar**
  **Maharashtra (IN)**
- **Kashikar, Sanjay Purushottam**
  **Maharashtra (IN)**
- **Khisti, Ratnaprabha Sadashiv**
  **Maharashtra (IN)**
- **Shinde, Babanrao Mahadeo**
  **Maharashtra (IN)**
- **Shivaram, Swaminathan**
  **Maharashtra (IN)**

(74) Representative: **Skailes, Humphrey John et al**
**Frank B. Dehn & Co.,**
**European Patent Attorneys,**
**179 Queen Victoria Street**
**London EC4V 4EL (GB)**

(56) References cited:
**EP-A- 0 306 714** **US-A- 3 835 100**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 477 444 B1

## Description

This invention relates to the polymerization of acrylonitrile or methacrylonitrile and to novel reactive anionic initiators. These initiators can be used for the polymerization of vinyl monomers with controlled structure and molecular weight. In particular, they are useful for polymerising very reactive vinyl monomers such as nitrile bearing vinyl compounds.

Conventional methods of preparation of carbanion initiators consists of reacting alkali metals such as Li, Na and alkaline earth metals such as Mg and Zn with alkyl or aryl halides (viz. chloride, bromide and iodide). (M. Morton, Anionic Polymerization, Principles and Practice, Academic Press, New York, 1983).

Initiators prepared by the above conventional method can be used for polymerization of vinyl compounds. However, adequate control on polymerization rate and structures for alpha activated vinyl monomers are achievable only at temperatures below -50°C. This is due to the extremely high reactivity of the initiators (P. Rempp, E. Franta and J-E. Herz, Advances in Polymer Science, Volume 86, p.147, 1988).

Conventional process of initiator synthesis involves reaction or organic halides with metals, which is a biphasic solid-liquid reaction. This heterogeneous nature of the reaction needs longer time and higher temperature for quantitative yield. Furthermore, scope is limited with respect to control of reactivity of the initiators.

The existing methods for synthesis of carbanionic initiators cannot provide control over their reactivities. Hence their use in polymerization of vinyl monomers with a wide range of reactivities are limited.

The major disadvantages of the initiators prepared by the above said processes are :

a) The initiators are unstable at temperature above -20°C and thus need storage at low temperature under inert atmosphere.

b) The metallic counterions being highly polar do not provide wider choice of using common organic solvents.

c) The metallic counterions which will remain with the polymer are detrimental to the properties of the polymer and need cumbersome procedures of purification.

d) The initiators lack structural diversity and are therefore not useful for introducing functional groups in the polymer.

e) Metal containing initiators thus prepared do not enable adequate control on molecular weight and molecular weight distribution of the polymers obtained from highly reactive vinyl monomers such as those bearing nitrile or ester groups.

Carbanions can also be prepared from organic compounds bearing labile hydrogens (acidic hydrogen) by deprotonation using a strong base such as sodium hydroxide, sodium hydride or n-butyl-lithium. However, these metal containing carbanions are also beset with the same drawbacks described above.

More recently it has been reported that ammoinium salt of resonance stabilized carbanions are efficient initiators for the polymerisation of (meth)acrylic acid derivatives (EP 0 360 714 A1). The resonance stabilized carbanions were derived from strong acidic organic compounds such as malonic acid esters, acetylacetic acid esters, dicyanoalkanes, nitroalkanes and 9-substituted fluorenes. Polymers of alkylacrylate could be synthesized with good control of polymerisation (polydispersity index of 1.1-1.3) using these initiators. Although acrylonitrile was reported to be polymerised using such initiators, the control of polymerisation was not demonstrated. However, the organic compounds described in this reference are extensively branched, contain only one tertiary hydrogen, the other substituents being strong electron withdrawing (such as cyano, nitro or ester) and thus form highly stabilized carbanions. These compounds have been known in the literature to be very acidic (pKa's being less than 10-11, measured in water at 25°C) (J.R. Jones, Ionization of Carbon Acids, Academic Press, New York, 1973, p.63) and thus easy to form salts with bases such as quaternary ammonium hydroxides (A Brand Strom, U. Junggren Acta. Chem. Scand, Vol.25, p.1469, 1971).

However, the prior art does not disclose the preparation of stable carbanions from simple organic compounds containing primary and secondary hydrogen atoms and their utility as polymerization initiators. These carbanions have been reported in the literature to lead to complicated side reactions such as proton transfer, undesired condensations between reacting species and competing 1,2 and 1,4 additions with unsaturated vinyl monomers containing $\alpha$-activating groups like carbonyl, nitrile etc. Carbanions from less acidic organic compounds can be generated using strong bases such as n-BuLi, lithium diisopropylamide etc. at a temperature of -50°C or below. The carbanions so generated are also stable only at these low temperatures (J.C. Stowell, Carbanions in Organic Synthesis, John Wiley and Sons, New York, 1979, p.144).

For controlled polymerization of very reactive monomers such as acrylonitrile, methacrylonitrile etc. it is desirable to have more nucleophilic carbanionic initiators whose reactivity matches closely that of the propagating carbanion.

this can be achieved only by tailoring the carbanion stability through choice of appropriate substituent.

The main object of the present invention is to provide a process for polymerizing acrylonitrile or methacrylonitrile in which carbanions are generated from less acidic organic compounds bearing secondary and primary hydrogen atoms without the attendant side reactions and complications reported in the prior art for the synthesis of such carbanions. These carbanions have metal free counter cations. Since the reactivity of carbanions can be tailored by suitable alpha-functional groups, these initiators can provide a means of introducing functional groups as head groups in oligomers and polymers.

Accordingly, the present invention provides a process for the polymerization of acrylonitrile or methacrylonitrile in which the polymerization is carried out in the presence of an initiator at a temperature of 0° to 60°C to produce a polymer which has an Mn of 500-25000 and a polydispersity index of 1.0-2.0, and the initiator is a carbanion salt containing a carbanion (A) and a cations (B), where:

A is (1) Ar $(R_1)(R_2)C^-$ or (2) $CN(R_1)(R_2)C^-$, where:

when A is a group (1), Ar is a phenyl or 1,3-$\Delta^2$-oxazoline group; and
$R_1$ and $R_2$ are both hydrogen atoms (when Ar is 1,3-oxazoline); or $R_1$ is a $-COOR_7$ (where $R_7$ is $C_{1-4}$ alkyl), cyano, 1,3-$\Delta^2$-oxazoline or N,N-dimethylamido group and $R_2$ is a hydrogen atom or is a group as just defined for $R_1$; or $R_1$, Ar and the carbon atom together form a dihydroisoquinolinyl group and $R_2$ is a cyano group or
when A is a group (2), $R_1$ is $-COOR_7$ $R_7$ is ethyl and $R_2$ is a hydrogen atom; and
B is $NR_3R_4R_5R_6$, where $R_3$, $R_4$, $R_5$, and $R_6$ (which may be the same or different) are hydrocarbyl groups of from 1 to 16 (e.g. 3-16) carbon atoms (no more than one of said groups being aryl or aralkyl), or two of said groups together with the nitrogen atom to which they are bound form a heterocyclic ring of 5 to 7 atoms, the total number of carbon atoms in $R_3$, $R_4$, $R_5$, and $R_6$ being no more than 50 (e.g. 12-50).

The carbanions are resonance stabilised by the presence of Ar (or CN) and $R_1$ groups.

$R_1$ is preferably an alpha-activating groups such as -COOR7, cyano or 1,3-$\Delta^2$-oxazoline.

In the cation B, the hydrocarbyl groups may for example be $C_{3-16}$ alkyl, $C_{5-8}$ cycloalkyl, phenyl $(C_{1-4})$ alkyl or phenyl groups.

The initiators used in the invention can be prepared by reacting a labile hydrogen containing compound A-H with a base B-OH, where A and B are as defined above. The compound A-H may for example be 2-benzyl-1,3-$\Delta^2$-oxazoline, diethylphenylmalonate, methylphenylacetate, 1-cyanaodiphyroisoquiniline, N,N-dimethyl phenylacetamide, bis (N,N-dimethylamide) phenyl methane, phenylmalonitrile or phenylacetonitrile. The base is generally one which is soluble in organic solvents, and may for example be tetra-n-butyl ammonium hydroxide or benzyl tri-n-butyl ammonium hydroxide. The reaction may be carried out under an inert atmosphere at a temperature range of +50° to +90°C.

The inert atmosphere may be achieved using purified dry nitrogen argon gas and the like.

The reaction may preferably be effected in the presence of organic solvents such as benzene, toluene, tetrahydrofuran.

The reaction mixture may be agitated and may be held at constant temperature for a minimum period of 3 hours. The solvent may be distilled out to give initiator which can be isolated either as an oil or an solid and stored under suitable conditions.

Certain of the initiators are new, these being the compounds as defined above where:

$R_1$ and $R_2$ are both hydrogen atoms (when Ar is 1,3-oxazoline); or $R_1$ is a 1,3-$\Delta^2$-oxazoline and $R_2$ is a hydrogen atom (when Ar is phenyl); or A is 1-cyanodihydroisoquinoline.

The following examples show the preparation of initiators used the invention.

Example 1

Preparation of Initiator from 2-Benzyl-1,3-$\Delta^2$-oxazoline: To a three necked 100 mL round bottom flask fitted with a thermometer, nitrogen inlet, rubber septum and magnetic needle, 2.5 mmol of 2-benzyl-1,3-oxazoline and 50 mL dry tetrahydrofuran (THF) were added with a hypodermic syringe under an atmosphere of dry nitrogen. While stirring, 2.5 mmol of tetrabutylammonium hydroxide (20% solution in toluene/methanol) was added. The reaction mixture was stirred for ½ hour at room temperature and the temperature was slowly raised to +50°C and maintained for 3 hours at that temperature. Appearance of a deep red colored solution is indicative of the carbanion formation. The reaction mixture was cooled to room temperature, the solvent was evaporated under vacuum and the product was dried at 40°C under vacuum. The carbanion salt (tetra-n-butyl ammonium 2-benzyl-1,3-$\Delta^2$-oxazoline) thus obtained is a deep red oily residue which has desired spectral features.

Example 2

Preparation of Initiator from Diethylphenylmalonate : To a three necked 100 mL round bottom flask fitted with a argon inlet, dropping funnel, distillation unit and magnetic needle, was added 7.7 mmoles of tetrabutylammonium hydroxide (20% solution in toluene/methanol) in 40 mL of dry toluene and heated to +75°C to +85°C. Diethylphenylmalonate (9.3 mmoles) was slowly added. Simultaneously an azeotrope of toluene-water was distilled out slowly over a period of 3 hours. After all toluene had distilled over, the flask was cooled to room temperature slowly. A solid product separated out, which was repeatedly washed with dry hexane. The initiator (tetra-n-butyl ammonium diethylphenyl malonate) thus formed was dried in vacuum and stored at room temperature under nitrogen or argon.

Example 3

Preparation of Initiator from Methylphenylacetate: In a similar apparatus as described in example 2, 7.7 mmoles of tetrabutylammonium hydroxide (20% solution in toluene/methanol) in 40 ml of dry toluene was taken and heated to +75°C to +85°C. Methylphenylacetate (9.0 mmoles) was slowly added. Simultaneously an azeotrope of toluene-water was distilled out slowly over a period of 3 hours. After all toluene had distilled over, the flask was cooled to room temperature slowly. A solid product separated out, which was repeatedly washed with dry hexane. The initiator (tetra-n-butyl ammonium methylphenylacetate) thus formed was dried in vacuum and stored at room temperature under nitrogen or argon.

Example 4

Preparation of Initiator from Ethylcyanoacetate: In a similar apparatus as described in example 2, 7.7 mmoles of tetra-n-butyl ammonium hydroxide (20% solution in toluene/methanol) in 40 ml of dry toluene and heated to +75°C to +85°C. Ethylcyanoacetate (9.4 mmoles) was slowly added. Simultaneously an azeotrope of toluene-water was distilled out slowly over a period of 3 hours. After all toluene had distilled over, the flask was cooled to room temperature slowly. A solid product separated out, which was repeatedly washed with dry hexane. The initiator (tetra-n-butyl ammonium ethylcyanoacetate) thus formed was dried in vacuum and stored at room temperature under nitrogen or argon.

Example 5

Preparation of Initiator from 2-Methyl-1,3-$\Delta^2$-oxazoline: In a similar experimental set described in example 1, 10 mmoles of 2-methyl-1,3-oxazoline and 50 ml dry tetrahydrofuran (THF) were added with hypodermic syringe under an atmosphere of dry $N_2$. While stirring 10 mmoles of tetra-n-butyl ammonium hydroxide (20% solution in toluene/methanol) was added. The reaction mixture was stirred for ½ hour at room temperature and the temperature was slowly raised to 50°C and maintained for 8 hours at that temperature. Appearance of faint yellow coloured solution is indicative of the carbanion formation. The reaction mixture was cooled to room temperature, the solvent was evaporated under vacuum and the product was dried at 40°C under vacuum. The carbanion salt (tetra-n-butyl ammonium 2-methyl-1,3-$\Delta^2$-oxazoline) obtained is a faint yellow oily residue which had the desired spectral features.

Example 6

Preparation of Initiator from N-benzoxy-1-cyano-dihydroisoquinoline: A similar experimental apparatus was set up as described in example 1, mmoles of N-benzoxy-1-cyano-dihydroisoquinoline and 50 ml dry tetrahydrofuran (THF) were added with a hypodermic syringe under an atmosphere of dry nitrogen. While stirring 5 mmoles of tetra-n-butyl ammonium hydroxide (20% solution in toluene/methanol) was added. The reaction mixture was stirred for ½ hour at room temperature and the temperature was slowly raised to +50°C and maintained for 3 hours at that temperature. Appearance of a deep red coloured solution in indicative of the carbanion formation. The reaction mixture was cooled to room temperature, the solvent was evaporated under vacuum and the product was dried at +40°C under vacuum. The carbanion salt (tetra-n-butyl ammonium-N-benzoxy-1-cyano-dihydro isoquinoline obtained is a deep red coloured oily residue.

The formulae of the carbanions in the above examples are:

Example 1

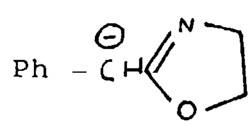

Example 2 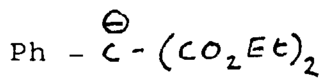

Example 3 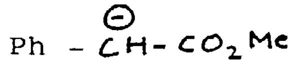

Example 4 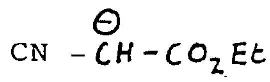

Example 5 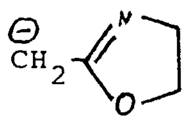

Example 6 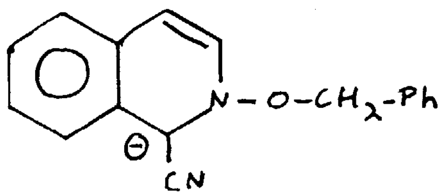

The main advantages of the present invention are :

a) The initiators can be prepared over a temperature range of +50°C to +90°C.

b) The initiators which are in the form of solids or liquids are isolable and can be conveniently stored at ambient temperature. The initiators can also be prepared in two phase systems containing of an organic and an aqueous phase.

c) They are free from metal ions.

d) Several types of functional groups can be incorporated by using a range of readily available organic compounds as starting materials. These enable the synthesis of a wide range of polymers with functional groups.

e) It is possible to vary the reactivity of the initiators over a wide range by the appropriate choice of substituents in the starting material.

As indicated above, the initiators can be used to prepare polyacrylonitrile, polymethacrylonitrile and the like.

High molecular weight polyacrylonitrile are fiber forming polymers. Polymers containing >90% acrylonitrile are commonly referred to as acrylic fibers and are produced commercially. Chemically modified polyacrylonitrile have diverse commercial applications in areas such as dispersants and flocculents in water treatment, oil well chemicals for enhanced oil recovery and as speciality polymers for mineral beneficiation, surface coatings, and lubricant oil additives. Oligomeric polyacrylonitrile and their modified derivatives find applications as synthetic thickening agents for printing inks in textile industries. Polyacrylonitrile are also commercially important precursor fibers for the manufacture of carbon fibers useful as components of advanced materials. Polymers bearing methacrylonitrile groups have commercially useful barrier properties and are useful in packaging applications.

Anionic polymerization of acrylic monomers can be initiated by (a) organolithium/organomagnesium or organosodium compounds (b) using ketene silyl acetals as initiators in the presence of a fluoride, bifluoride, cyanide or azides as catalysts or (c) using metal free carbon nucleophiles as initiators.

Method described at (a) above has been successfully applied for the controlled synthesis of styrene and diene polymers. However, application of the process to acrylonitrile does not lead to desired results [A. Ottolenghi and A. Zilkha, J. Polym. Sci., Part A 1, 687 (1963); 3643 (1963)]. Depending on catalyst concentration, homogeneous anionic

polymerization of acrylonitrile by butyllithium showed spontaneous termination by chain transfer to monomer or intramolecular cyclization reactions of the growing chain end. Molecular weight control by control of initiator concentration could be achieved only at very low initiator concentrations.

Method described at (b) above when applied to acrylonitrile led to uncontrolled polymerization leading to gel formation. Only at -50°C the polymerization could be controlled. Nevertheless, polydispersity was broad (3.79) [D.Y. Sogah, W.R. Hertler, D.W. Webster and G.M. Cohen, Macromomolecules, 20 1473 (1987)].

Method described at (c) above though effective for acrylic ester monomers, does not appear to be applicable to controlled polymerization of very reactive nitrile bearing vinyl monomers [M.T. Reetz, Angew. Chem. Int., Ed. Engl. 27 (7), 994 (1988)].

Thus, these prior art processes are not satisfactory for the preparation of nitrile bearing polymers in a controlled manner.

Polymers and copolymers of acrylonitrile are generally produced by free radical polymerization processes using redox type initiators in aqueous medium. Such processes are known to be deficient in the following respects:

a) Polymerization is normally conducted in the temperature range of 70° - 90°C.

b) The initiators used normally contain metals. Since metallic residues are detrimental to polymer, washing of polymer and removal of metal residues is essential.

c) Molecular weight control normally requires addition of external agents, called chain transfer agents.

d) Polymers produced have broad molecular weight distribution; polydispersity values generally are in the range of 2.5 to 3.0.

e) It is difficult to produce by this method, polymers of precisely controlled molecular weights.

f) The method is not suitable for introduction of functional groups at the polymer chain end.

Most of the drawbacks of the free radical processes can be obviated by the use of anionic polymerization process. Anionic processes generally afford more control on polymerization, use relatively lower temperatures and produce narrow molecular weight distribution polymers. Control of molecular weight is possible by adjusting the initiator concentrations. However, the present state of the art of anionic polymerization of acrylonitrile or acrylic acid esters as monomers is beset with the following drawbacks :

a) The reactions initiated by conventional anionic intiators such as organolithium, organomagnesium compounds require low reaction temperatures (-50° to -75°C) for adequate control of reaction.

b) Reactions at higher temperatures become progressively less controllable, leading to unwanted side reactions and resulting in polymers with broad molecular weight distributions.

c) Commonly used initiators enter into wasteful side reactions with nitrile group in acrylonitrile. Recently a new polymerization technique has been discovered, which offers advantages in polymerizing acrylic type monomers over conventional anionic polymerization processes. This technique, termed in the literature as "Group Transfer Polymerization (GTP)" involves the use of initiators containing silicon atoms (e.g. 1-methoxy-2-methyl-1-trimethylsiloxy-1-propene) and a catalyst generally containing a fluoride ion (e.g. tetrabutyl ammonium fluoride). Whereas the use of this initiator/catalyst system with monomers such as acrylic acid esters leads to a well controlled/defined polymers with narrow molecular weight distribution, the extension of this system to acrylonitrile/methacrylonitrile has not met with success. These monomers are still too reactive for group transfer initiator/catalysts system and even at low temperatures, uncontrollable reactions occur leading to polymers with molecular weight distribution, as broad as 3.5 - 3.9.

More recently, a class of metal free carbanion initiators have been described in the literature for controlled polymerization of acrylic acid esters at room temperature. However, the literature does not reveal its applicability to the polymerization of nitrile bearing monomers (Sogah et al, Macromolecules, Vol. 20, P. 1483 (1987)).

An object of the present invention is (a) to provide a process for the polymerization of acrylonitrile/methacrylonitrile using metal free anionic initiators in solution or slurry; (b) to enable the polymerization to be conducted in the temperature range 0° to +60°C, with adequate control on polymerization (c) to enable the production of polymers with a wide range of molecular weights by mere control of initiator concentration and (d) to produce polymers with reasonably

narrow molecular weight distributions.

Acrylonitrile is a reactive vinyl monomer with high Q and e value (Q = 1.20, e = 0.60) and hence amenable to polymerization using anionic polymerization initiator. Conventional anionic initiators, like organolithium, organosodium or organomagnesium compounds react with both the double bond as well as the nitrile group, leading to a number of unwanted side reactions and products. The main object of the present invention is to provide an improved process for the preparation of nitrile containing polymers overcoming the above said drawbacks in the hitherto known process.

The resonance stabilized carbanions (initiators) of the inventors can provide a range of reactivities suitable for vinyl polymerization of activated monomers such as acrylonitrile/methacrylonitrile etc.

By appropriate adjustment of the monomer to initiator ratio, polymers with molecular weights in the range of 500 to 25,000 can be prepared. The molecular weight is controllable by adjustment of monomer to initiator ratio only indicating that the polymerization is free from unwanted side reactions such as chain transfer, termination, intramolecular cyclization etc.

Uptil now initiators as mentioned above have not been used for polymerization of nitrile bearing monomers. Even the initiators that are used in group transfer polymerization methods, a recently discovered method for controlled polymerization of acrylic ester monomers, fail to polymerize acrylonitrile with any degree of control at temperatures as low as -50°C (Macromolecules, Vol. 20, P. 1483 (1987)).

The inert atmosphere may be achieved using dry nitrogen or argon and the like. The reaction may preferably be effected in the presence of solvents such as tetrahydrofuran, dimethyl formamide and dimethyl acetamide.

The reaction mixture may be agitated and may be held at the constant temperature for the period of 2 hours, preferred time being 1 hour.

The resultant polymer may be either precipitated out as a slurry or kept as a homogenous solution depending on the use of solvents. The polymers prepared by the process have $\overline{M}n$ in the range of 500-25,000 and has a narrow polydispersity index in the range of 1.0 - 2.0. The yield of the polymer is almost quantitative.

The reaction tends to slow down or even stop when impurities with acidic hydrogen are present.

The process is described with reference to the following examples which should not be construed to limit the scope of the invention.

Example 7

Polymerization of Acrylonitrile using initiator (1) tetra-n-butyl ammonium 2-benzyl-1,3-$\Delta^2$-oxazoline Tetrahydrofuran:

A dry three necked 100 mL flask fitted with thermometer, nitrogen inlet, rubber septum and magnetic needle was charged with 30 mL dry THF under dry nitrogen atmosphere. To it 0.4 g (1 mmol) of the initiator 1 was added and the reactor was cooled to +2°C. Subsequently, 3.2g (60 mmol) of acrylonitrile was injected slowly. The temperature rose from +2°C to +6°C. After 15 minutes, the temperature came back to its original value of +2°C. The reaction was allowed to continue for 1 hour and was terminated by pouring into 150 mL of methanol. The solid mass was filtered and dried under vacuum giving 2.85g of poly(acrylonitrile) (89% conversion). Vapour pressure osmometry gave a $\overline{M}n$ of 2690 (calc value 3180).

Example 8

Polymerization of Acrylonitrile using Initiator tetra-n-butyl ammonium 2-benzyl-1,3-$\Delta^2$-oxazoline in N,N-dimethyl formamide:

A 100 mL three necked flask having accessories as in the case of example 7, was charged with 30 ml of dry DMF and 0.08g (0.2 mmol) of the initiator 1 and cooled to +2°C. To it, 4g (76 mmol) of acrylonitrile was added slowly under nitrogen. The temperature rose to +5°C. After 1 hour, the reaction was terminated by pouring into 150 ml methanol. The polymer was filtered off and dried under vacuum yielding 2.6g (65% conversion) of poly(acrylonitrile). The polydispersity index as determined by gel permeation chromatography was 1.7.

Example 9

Polymerization of Acrylonitrile using Initiator (2) tetra-n-butyl ammonium diethyl phenyl malonate in Tetrahydrofuran:

A dry 100 mL three necked flask equipped with magnetic needle, nitrogen/argon inlet tube, rubber septum and thermometer inlet, was charged with 15 mL of dry THF under argon atmosphere. To it 0.294 mmoles of the initiator 2 was added. Subsequently 3.2 g (60 mmoles) of acrylonitrile was added to flask at +28°C slowly. The reaction temperature spontaneously increased from +28°C to +43°C. After 1 hour the reaction was terminated by addition of acidified

methanol and precipitated by pouring into excess of methanol. The solid poly(acrylonitirle) polymer was filtered and dried in vacuum at +50°C to give 2.6 g polymer (81% conversion). The polydispersity index as measured by gel permeation chromatography was 1.16.

Example 10

Polymerization of Acrylonitrile using initiator tetra-n-butyl ammonium diethyl phenyl malonate in Dimethylacetamide :

In a similar apparatus as described in example 9, 3.6 g (68.4 mmoles) of acrylonitrile was added to 0.224 mmoles of initiator 2 in dimethylacetamide (20 mL) at +28°C. The reaction temperature rose from +28°C to +47°C. After 1 hour, the polymerization was terminated by addition of acidified methanol and precipitated by pouring into excess of methanol. The precipitated poly(acrylonitrile) was filtered, washed and dried in vacuum at +50°C to give 2.5 g polymer (70% conversion). The polydispersity index as measured by gel permeation chromatography was 1.82.

Example 11

Polymerization of Acrylonitrile using Initiator tetra-n-butyl ammonium diethyl phenyl malonate in Dimethyl formamide :

In a similar apparatus as described in example 9, 3.6 g (68.4 mmoles) of acrylonitrile was added to 0.115 mmole of Initiator 2 in DMF (20 mL) at +28°C. The temperature rose to +46°C Poly(acrylonitrile) was isolated as in example 10. Yield 2.2 g (60% conversion). The polydispersity index as measured by gel permeation chromatography was 1.90.

Example 12

Polymerization of Acrylonitrile using Initiator tetra-n-butyl ammonium diethyl phenyl malonate in Dimethyl formamide :

In a similar apparatus as described in example 9 , but using a 250 mL reaction flask, 18.5 g (349 mmoles) of acrylonitrile was added to 0.96 mmoles of initiator 2 in 100 ml DMF at +28°C. The reaction temperature increased to °57°C. After 1 hour, poly(acrylonitrile) was precipitated and isolated as before to give 16.0 g of poly(acrylonitrile) (86% conversion) The polydispersity index was measured by gel permeation chromatography was 1.72.

Example 13

Polymerization of Methacrylonitrile using Initiator tetra-n-butyl ammonium diethyl phenyl malonate in Tetrahydrofuran:

A dry 25 mL two necked flask equipped with magentic needle, nitrogen/argon inlet tube and rubber septum was charged with 3 mL of dry THF under argon atmosphere. To it 0.39 mmoles of the initiator 2 was added. Subsequently, 0.64 g (9.55 mmoles) of methacrylonitrile was added to the flask. The flask was held within the temperature range of +55°C to +60°C. After 6 hours the reaction was terminated by addition of acidified methanol. The poly(methacrylonitrile) was isolated as in example 10. Yield 0.698 g (95% conversion).

The main advantages of the process are:

a) The polymerization can be conducted conveniently in the temperature range 0° to +60°C.

b) The initiators are derived from readily available organic compounds.

c) The initiators are free from metal ion.

d) No additional catalysts are required as in the case of group transfer polymerization (GTP).

e) The structural diversity that can be built into the initiator is very large. This allows tailoring of initiator reactively to suit the monomers.

f) The initiators have reactive functional groups, which form the head group of polymers. Thus, a range of oligomers and polymers which have reactive groups at one end of the polymer chain is available by the use of this technique.

g) The molecular weight is controlled to a predetermined value by simply adjusting the initiator concentration.

h) The process affords polymers with a relatively narrow molecular weight distribution, the polydispersity values being lower than 2.0 which are unattainable by any of the hitherto known processes.

## Claims

**1.** A process for the polymerisation of acrylonitrile or methacrylonitrile in which the polymerisation is carried out in the presence of an initiator at a temperature of 0° to 60°C to produce a polymer which has an Mn of 500-25000 and a polydispersity index of 1.0-2.0, and the initiator is a carbanion salt containing a carbanion (A) and a cation (B), where:
A is (1) Ar $(R_1)(R_2)C^-$ or (2) $CN(R_1)(R_2)C^-$, where:

when A is a group (1), Ar is a phenyl or 1,3-$\Delta^2$-oxazoline group; and
$R_1$ and $R_2$ are both hydrogen atoms (when Ar is 1,3-oxazoline); or $R_1$ is a $-COOR_7$ (where $R_7$ is $C_{1-4}$ alkyl), cyano, 1,3-$\Delta^2$-oxazoline or N,N-dimethylamido group and $R_2$ is a hydrogen atom or is a group as just defined for $R_1$, or A is 1-cyanodihydroisoquinoline or
when A is a group (2), $R_1$ is $-COOR_7$ $R_7$ is ethyl and $R_2$ is a hydrogen atom; and
B is $NR_3R_4R_5R_6$, where $R_3$, $R_4$, $R_5$, and $R_6$ (which may be the same or different) are hydrocarbyl groups of from 1 to 16 carbon atoms (no more than one of said groups being aryl or aralkyl), or two of said groups together with the nitrogen atom to which they are bound form a heterocyclic ring of 5 to 7 atoms, the total number of carbon atoms in $R_3$, $R_4$, $R_5$, and $R_6$ being no more than 50.

**2.** A process as claimed in claim 1 in which the initiator is tetra-n-butylammonium 2-benzyl-1,3-$\Delta^2$ oxazoline.

**3.** A process as claimed in claim 1 in which the initiator is tetra-n-butyl ammonium diethylphenylmalonate.

**4.** A process as claimed in claim 1 in which the initiator is tetra-n-butyl ammonium methylphenylacetate.

**5.** A process as claimed in claim 1 in which the initiator is tetra-n-butyl ammonium ethylcyanoacetate.

**6.** A process as claimed in claim 1 in which the initiator is tetra-n-butylammonium 2-methyl-1,3-$\Delta^2$-oxazoline.

**7.** A process as claimed in claim 1 in which the initiator is tetra-n-butylammonium N-benzoxy-1-cyano-dihydroisoquinoline.

**8.** An anionic initiator as defined in claim 1 where:

A is a group (1), Ar is a phenyl or 1,3-oxazoline-$\Delta^2$-group, and
$R_1$ and $R_2$ are both hydrogen atoms (when Ar is 1,3-$\Delta^2$ oxazoline); or $R_1$ is a 1,3-$\Delta^2$-oxazoline and $R_2$ is a hydrogen atom (when Ar is phenyl); or A is 1-cyanodihydroisoquinoline.

**9.** An initiator as claimed in claim 8 and as defined in claim 2, claim 6 or claim 7.

## Patentansprüche

**1.** Verfahren zur Polymerisation von Acrylnitril oder Methacrylnitril, in dem die Polymerisation in Gegenwart eines Starters bei einer Temperatur von 0° bis 60°C ausgeführt wird, unter Herstellung eines Polymers, das ein $\overline{M}n$ von 500-25000 und einen Polydispersitätsindex von 1,0-2,0 aufweist und der Starter ein Carbanionsalz, enthaltend ein Carbanion (A) und ein Kation (B), darstellt, worin:
A (1) $Ar(R_1)(R_2)C^-$ oder (2) $CN(R_1)(R_2)C^-$ darstellt, worin:

wenn A eine Gruppe (1) darstellt, Ar eine Phenyl- oder 1,3-$\Delta^2$-Oxazolingruppe darstellt; und
$R_1$ und $R_2$ beide Wasserstoffatome darstellen (wenn Ar 1,3-Oxazolin darstellt); oder $R_1$ eine Gruppe $-COOR_7$ (worin $R_7$ $C_{1-4}$-Alkyl darstellt), Cyano-, 1,3-$\Delta^2$-Oxazolin- oder N,N-Dimethylamidogruppe darstellt und $R_2$ ein Wasserstoffatom oder eine Gruppe wie gerade für $R_1$ definiert darstellt; oder A 1-Cyanodihydroisochinolin darstellt oder
wenn A eine Gruppe (2) darstellt, $R_1$ $-COOR_7$, $R_7$ Ethyl und $R_2$ ein Wasserstoffatom darstellt; und

B $NR_3R_4R_5R_6$ darstellt, wenn $R_3$, $R_4$, $R_5$ und $R_6$ (die gleich oder verschieden sein können) Kohlenwasserstoffgruppen mit 1 bis 16 Kohlenstoffatomen darstellen (wobei nicht mehr als eine der Gruppen Aryl oder Aralkyl ist), oder zwei der Gruppen zusammen mit dem Stickstoffatom, an das sie gebunden sind, einen heterocyclischen Ring mit 5 bis 7 Kohlenstoffatomen bilden, wobei die Anzahl der Kohlenstoffatome in $R_3$, $R_4$, und $R_6$ insgesamt nicht mehr als 50 ist.

**2.** Verfahren nach Anspruch 1, in dem der Starter Tetra-n-butylammonium-2-benzyl-1,3-$\Delta^2$-oxazolin darstellt.

**3.** Verfahren nach Anspruch 1, in dem der Starter Tetra-n-butylammoniumdiethylphenylmalonat darstellt.

**4.** Verfahren nach Anspruch 1, in dem der Starter Tetra-n-butylammoniummethylphenylacetat darstellt.

**5.** Verfahren nach Anspruch 1, in dem der Starter Tetra-n-butylammoniumethylcyanoacetat darstellt.

**6.** Verfahren nach Anspruch 1, in dem der Starter Tetra-n-butylammonium-2-methyl-1,3-$\Delta^2$-oxazolin darstellt.

**7.** Verfahren nach Anspruch 1, in dem der Starter Tetra-n-butylammonium-N-benzoxy-1-cyanodihydroisochinolin darstellt.

**8.** Anioinischer Starter nach Anspruch 1, worin:

A eine Gruppe (1) darstellt, Ar eine Phenyl- oder 1,3-$\Delta^2$-Oxazolingruppe darstellt, und
$R_1$ und $R_2$ beide Wasserstoffatome darstellen (wenn Ar 1,3-$\Delta^2$-Oxazolin darstellt); oder $R_1$ 1,3-$\Delta^2$-Oxazolin darstellt und $R_2$ ein Wasserstoffatom darstellt (wenn Ar Phenyl darstellt) ; oder A 1-Cyanodihydroisochinolin darstellt.

**9.** Starter nach Anspruch 8 und wie in Anspruch 2, Anspruch 6 oder Anspruch 7 definiert.

## Revendications

**1.** Procédé pour la polymérisation d'un acrylonitrile ou méthacrylonitrile dans lequel la polymérisation est effectuée en présence d'un initiateur à une température allant de 0 °C à 60 °C pour produire un polymère qui a un poids moléculaire en nombre Mn de 500 à 25 000 et un indice de polydispersion de 1,0 à 2,0, et dans lequel l'initiateur est un sel de carbanion contenant un carbanion (A) et un cation (B), où :
A est (1) Ar ($R_1$) ($R_2$) $C^-$ ou (2) CN ($R_1$) ($R_2$) $C^-$, où :

lorsque A est un groupe (1), Ar est un groupe phényle ou 1,3-$\Delta^2$-oxazoline ; et
$R_1$ et R2 sont tout deux un atome d'hydrogène (lorsque Ar est le groupe 1,3-oxazoline) ; ou $R_1$ est un groupe $-COOR_7$ (où $R_7$ est un alkyle en $C_1$-$C_4$), cyano, 1,3-$\Delta^2$-oxazoline ou N,N-diméthylamido et $R_2$ est un atome d'hydrogène ou un groupe tel que défini juste avant pour $R_1$ ; ou A est un groupe 1-cyanodihydroisoquinoléine ou
lorsque A est un groupe (2), $R_1$ est un groupe $-COOR_7$, $R_7$ étant le groupe éthyle et $R_2$ est un atome d'hydrogène ; et
B est $NR_3R_4R_5R_6$, où $R_3$, $R_4$, $R_5$, et $R_6$ (qui peuvent être identiques ou différents) sont des groupes hydrocarbyles contenant 1 à 16 atomes de carbone (l'un au plus de ces groupes étant un groupe aryle ou aralkyle), ou deux de ces groupes pris conjointement avec l'atome d'azote auquel ils sont liés forment un hétérocycle à 5 ou 7 atomes, le nombre total d'atomes de carbone dans $R_3$, $R_4$, $R_5$ et $R_6$ n'étant pas supérieur à 50.

**2.** Procédé selon la revendication 1 dans lequel l'initiateur est le tétra-n-butylammonium de 2-benzyl-1,3-$\Delta^2$-oxazoline.

**3.** Procédé selon la revendication 1 dans lequel l'initiateur est le tétra-n-butyl ammonium de phénylmalonate de diéthyle.

**4.** Procédé selon la revendication 1 dans lequel l'initiateur est le tétra-n-butyl ammonium de phénylacétate de méthyle.

5. Procédé selon la revendication 1 dans lequel l'initiateur est le tétra-n-butyl ammonium de cyanoacétate d'éthyle.

6. Procédé selon la revendication 1 dans lequel l'initiateur est le tétra-n-butylammonium de 2-méthyl-1,3-$\Delta^2$-oxazoline.

7. Procédé selon la revendication 1 dans lequel l'initiateur est le tétra-n-butylammonium de N-benzoxy-1 -cyano-dihydroisoquinoléine.

8. Initiateur anionique tel que défini selon la revendication 1 dans lequel :

   A est un groupe (1), Ar est un groupe phényle ou 1,3-$\Delta^2$-oxazoline, et
   $R_1$ et R2 sont tout deux un atome d'hydrogène (lorsque Ar est le 1,3-$\Delta^2$-oxazoline) ; ou $R_1$ est le 1,3-$\Delta^2$-oxazoline et $R_2$ est un atome d'hydrogène (lorsque Ar est un groupe phényle) ; ou A est le 1-cyanodihydroisoquinoléine.

9. Initiateur selon la revendication 8 et tel que défini selon la revendication 2, la revendication 6 ou la revendication 7.